Europäisches Patentamt

European Patent Office

Office européen des brevets

(11) Veröffentlichungsnummer: **0 287 830**
**A1**

## EUROPÄISCHE PATENTANMELDUNG

(21) Anmeldenummer: 88104696.5

(22) Anmeldetag: 24.03.88

(51) Int. Cl.4 **C07C 69/24 , A61K 47/00 ,**
**A61K 31/23**

(30) Priorität: 31.03.87 DE 3710770

(43) Veröffentlichungstag der Anmeldung:
26.10.88 Patentblatt 88/43

(84) Benannte Vertragsstaaten:
**AT BE CH DE ES FR GB GR IT LI LU NL SE**

(71) Anmelder: **BEHRINGWERKE**
**Aktiengesellschaft**
**Postfach 1140**
**D-3550 Marburg 1(DE)**

(72) Erfinder: **Bernhardt, Dieter, Dr.**
**Goldbergstrasse 18A**
**D-3553 Cölbe(DE)**
Erfinder: **Kolar, Cenek, Dr.**
**Deutschhausstrasse 20**
**D-3550 Marburg(DE)**

(74) Vertreter: **Becker, Heinrich Karl Engelbert, Dr.**
**et al**
**HOECHST AKTIENGESELLSCHAFT Central**
**Patent Department P.O. Box 80 03 20**
**D-6230 Frankfurt am Main 80(DE)**

(54) **Neue Alkansäurealkylester, Verfahren zu ihrer Herstellung und Verwendung von wässrigen Emulsionen dieser Ester in Vaccinen.**

(57) Es werden neue Alkansäurealkylester der Formel I

$R^1$-CO-O-$R^2$      I

worin
$R^1$ eine lineare Alkylgruppe mit 5, 7, 9, 11, 13, 15 oder 17 Kohlenstoffatomen und
$R^2$ eine lineare Alkylgruppe mit 6, 8, 10, 12, 14, 16 oder 18 Kohlenstoffatomen darstellen, ein Verfahren zu ihrer Herstellung und die Verwendung von Alkylsäurealkylestern in wässriger Emulsion zur Adjuvierung von Antigenen oder zur Modulierung einer Immunantwort.

## Neue Alkansäurealkylester, Verfahren zu ihrer Herstellung und Verwendung von wässrigen Emulsionen dieser Ester in Vaccinen

Die Erfindung betrifft neue Alkansäurealkylester, Verfahren zu ihrer Herstellung und die Verwendung von Alkansäurealkylestern in wässriger Emulsion zur Adjuvierung von Antigenen oder zur Modulierung einer Immunantwort.

Die neuen Alkansäurealkylester entsprechen der Formel I

$$R^1\text{-}CO\text{-}O\text{-}R^2 \quad \text{I}$$

worin

$R^1$ eine lineare Alkylgruppe mit 5, 7, 9, 11, 13, 15 oder 17 Kohlenstoffatomen und

$R^2$ eine lineare Alkylgruppe mit 6, 8, 10, 12, 14, 16 oder 18 Kohlenstoffatomen darstellen.

Für die Herstellung von Vaccinen und Immunmodulatoren werden physiologisch verträgliche und vorzugsweise abbaubare chemische Verbindungen gebraucht, die die immunogene Wirkung von Antigenen unterstützen und/oder die Immunabwehr des Organismus verstärken.

In Adv. Tuberc. Res. 7, 130 (1956) sowie Vaccine, Vol. 3, 137 (1985) sind Öl-in-Wasser und Wasser-in-Öl-Emulsionen beschrieben, die die Fähigkeit besitzen, die immunogene Wirkung von Antigenen zu steigern. Die in diesen Emulsionen verwendeten Paraffin-Öle sind physiologisch nicht oder nur schwer abbaubar.

In Immunology 44, 187 (1981) ist die adjuvante Aktivität von Fettsäureethyl-, isopropyl-und isobutylestern beschrieben. Die adjuvante Aktivität solcher Ester erscheint hiernach erst dann, wenn der Säureanteil 16 oder mehr Kohlenstoffatome besitzt.

Es wurde überraschenderweise gefunden, daß Hexansäurehexylester als Bestandteil einer Emulsion vom Öl-in-Wasser-Typ immunstimulatorische und adjuvante Wirkung im Mäusetestmodell besitzt, und daß dessen Emulsionen im Meerschweinchentestmodell sehr gut verträglich sind.

Der vorliegenden Erfindung liegt deshalb die Aufgabe zugrunde, neue Alkansäurealkylester herzustellen, diese mit Hilfe von Emulgatoren in eine Emulsion vom Öl-in-Wasser-Typ zu überführen und als Adjuvans-und Immunmodulator-Formulierungen zu verwenden.

Gelöst wird diese Aufgabe durch die Herstellung von Verbindungen der Formel I und den angegebenen Definitionen, ihre Überführung in physiologisch verträgliche Emulsionen sowie ihre Prüfung im Säugetiermodell auf Verträglichkeit und Wirksamkeit zur Adjuvierung von Vaccinen und Stimulierung des Immunsystems.

Die Erfindung is in den Patentansprüchen definiert. Weitere Ausgestaltungen sind im folgenden beschrieben.

Die Alkansäurealkylester werden nach in der organischen Chemie bekannten Verfahren zur Darstellung von Estern wie z.B. aus einem Alkanoylhalogenid besonders einem Alkanoylchlorid und einem Alkohol in Gegenwart einer Base wie Pyridin, Triethylamin und eines Lösungsmittels wie Dichlormethan oder Ether hergestellt.

Gegenstand der Erfindung ist daher auch ein Verfahren zur Herstellung einer Verbindung der Formel I mit den angegebenen Definitionen, dadurch gekennzeichnet, daß eine Verbindung der Formel II

$$R^1 \, CO \, X \quad \text{II}$$

worin X ein die CO-Gruppe aktivierendes Atom oder eine solche Gruppe ist und $R^1$ die zur Formel I angegebene Bedeutung hat,
mit einer Verbindung der Formel III

$$R^2OH \quad \text{III}$$

worin $R^2$ die zur Formel I angegebene Bedeutung hat, zu einer Verbindung der Formel I umgesetzt wird.

Für den erfindungsgemäßen Zweck geeignete, stabile Emulsionen werden hergestellt, indem eine oder mehrere Verbindungen der Formel I, ein oder mehrere Emulgatoren und Wasser oder eine physiologische Pufferlösung und/oder eine Antigenlösung emulgiert werden.

Geeignete Emulgatoren, ihre chemischen und physikalischen Eigenschaften und ihre Verwendung sind in der Arzneiformellehre. Wissenschaftliche Verlagsgesellschaft mbH Stuttgart, 1982 (157-164) beschrieben.

Geeignet sind beispielsweise übliche Emulgatoren wie Lecithine, Saponine, Fettsäureester von Fettalkoholen, Zuckern und Zuckeralkoholen, Ethylenoxydkondensaten oder Glycerinkondensaten, wobei diese pharmakologisch und physiologisch verträglich sein müssen.

Eine erfindungsgemäße Emulsion enthält

    a) 0.1-10 ml/100 ml eines oder mehrerer Alkansäurealkylester,

    b) 0.01-40 ml/100 ml eines oder mehrerer Emulgatoren,

    c) Wasser oder eine Pufferlösung

und/oder

    d) 50-98 ml/100 ml einer oder mehrerer Antigenlösungen.

Die Ester sind flüssig oder können durch geringes Erwärmen verflüssigt werden.

Solche Emulsionen, die ein Antigen enthalten, können als Adjuvantien und solche, die kein Antigen enthalten, als Immunstimulantien in Wirbeltieren, vorzugsweise in Nutztieren und Haustieren verwendet werden. Eine Verwendung im Menschen scheint aufgrund von Untersuchungen am Affen möglich.

Auf diese Weise adjuvierbare Antigene sind vorzugsweise Virus-, Bakterien-, Zell-oder Proteinantigen oder gentechnisch hergestellte Antigen, besonders IBR (Infectious Bovine Rhinotracheitis)-Antigen, BAV-3 (Bovines Adeno-Virus, Serotyp 3), MKS (Maul-und Klauenseuche)-Virus, Aujeszkyvirus, PI$_3$ (Parainfluenza, Typ 3)-Virus oder RSV (Rous Sarcoma Virus).

In den folgenden Beispielen wird die vorliegende Erfindung näher beschrieben, ohne sie hierdurch einzuschränken.

## BEISPIELE

Beispiel 1 Herstellung von Alkansäurealkylestern

Hexansäureoctylester (Verbindung 1)

100 g (0.768 mol) Octanol wurden in 400 ml Dichlormethan gelöst. Nach der Zugabe von 300 ml Pyridin wurde die Lösung auf 0°C abgekühlt. Anschließend wurden 110.24 g (0.819 mol) Hexansäurechlorid gelöst in 300 ml Dichlormethan bei 0°C unter Rühren zugetropft. Die Reaktionslösung wurde dann 14 h bei Raumtemperatur gerührt. Der Verlauf der Umsetzung wurde dünnschichtchromatographisch verfolgt (Laufmittel: Hexan/Diisopropylether 3:1; Rf-Wert des Reaktionsproduktes 0.78). Der Reaktionsansatz wurde im Wasserstrahlpumpenvakuum (i.Vac.) zum Sirup eingedampft. Das in Petrolether gelöste Produkt wurde mit Wasser, verdünnter Salzsäure ausgewaschen. Das nach Abdampfen i.Vac. erhaltene Produkt wurde im Hochvakuum (0.015 mbar) destilliert.

Ausbeute: 148 g (Verbindung 1)

S.p. = 86°C bei 0.015 mbar

$^{13}$C-NMR (90 MHz, CDCl$_3$, δ): 171.48 (COO), 65.12 (CH$_2$O), 32.5 bis 21.45 (10$^{\times}$CH$_2$), 14,62 (2$^{\times}$CH$_3$)

Die chemische Reinheit der Verbindung 1 wurde gaschromatographisch gesichert.

Beispiel 2

Entsprechend Beispiel 1 wurden folgende Alkansäurealkylester hergestellt:

CH$_3$-(CH$_2$)$_n$-COO-(CH$_2$)$_m$-CH$_3$

3

## Tabelle 1:

| Verbindungs-Nr. | n+2 | m+1 | S.p. (°C) | |
|---|---|---|---|---|
| 2 | 6 | 6 | 81 | (0.1 mbar) |
| 3 | 6 | 10 | 101 | (0.004 mbar) |
| 4 | 6 | 12 | 118 | (0.003 mbar) |
| 5 | 8 | 6 | 85 | (0.015 mbar) |
| 6 | 8 | 8 | 104 | (0.003 mbar) |
| 7 | 8 | 10 | 124 | (0.003 mbar) |
| 8 | 8 | 12 | | |
| 9 | 10 | 6 | 310 | |
| 10 | 10 | 8 | 333 | |
| 11 | 10 | 10 | 355 | |
| 12 | 10 | 12 | 376 | |
| 13 | 12 | 6 | | |
| 14 | 12 | 8 | | |
| 15 | 12 | 10 | | |
| 16 | 12 | 12 | 393 | |
| 17 | 16 | 6 | | |
| 18 | 16 | 8 | | |
| 19 | 16 | 10 | | |
| 20 | 16 | 12 | | |
| 21 | 16 | 14 | 436 | |
| 22 | 16 | 16 | 451 | |
| 23 | 18 | 6 | 449 | |

## Beispiele zur Herstellung und Adjuvierung von Vaccinen

Die verwendeten Mengen sind in ml/100 ml wäßriger Pufferlösung oder Suspension angegeben.

## Beispiel 3

Mit inaktiviertem IBR (Infectious Bovine Rhinotracheitis)-Antigen

Es wurden Vaccinen der angegebenen Zusammensetzung mit IBR-Virus als Antigen hergestellt. IBR-Virus wird in Gewebekulturen, beispielsweise Kälbernierenkulturen, vermehrt. Nach völliger Zellzerstörung

4

wird das Virus geerntet, zentrifugiert, filtriert und mit Formalin 1:1000 bei 37°C 24 Stunden inaktiviert. Das inaktivierte Virus ist das Vaccineantigen und wird in der angegebenen Reihenfolge dem Adjuvans unter Ultraturaxbehandlung zugegeben und an Meerschweinchen und Hairless-Mäusen geprüft. Vaccine 1:

Decansäuredodecylester        4.0
[R]Tween 81 Emulgator        1.0
Entschäumer Bayer E61E 1881/11        0.5
IBR-Antigen        94.5

Vaccine 2:
[R]Tween 81        1.0
Entschäumer Bayer E61E 1881/11        0.5
IBR-Antigen        98.5

Vaccine 3:
IBR-Antigen        100.0

Vaccine 4:
Palmitinsäurehexadecylester        1.5
[R]Tween 81        0.5
IBR-Antigen        98.0

Vaccine 5:
Octansäureoxtylester        5.0
[R]Tween 81        1.0
Entschäumer Bayer E61 E 1881/11        0.5
IBR-Antigen        94.5

Vaccine 6:
Decansäuredodecylester        5.0
[R]Tween 81        1.0
Entschäumer Bayer E 61 E 1881/11        0.5
IBR-Antigen        93.0
Saponin 10 ml/100 ml Lösung        0.5

Vaccine 7:
Al(OH)$_3$ 2 g/100 ml Suspension        39.0
IBR-Antigen        61.0

Prüfung auf Verträglichkeit:

Diese Vaccinen wurden am Meerschweinchen und Hairless-Mäusen auf Verträglichkeit getestet. Mit jeder Vaccine wurden jeweils 3 Meerschweinchen intraplantar und 3 Hairless-Mäuse s.c. vacciniert. Die Dosis pro Tier betrug 0.2 ml. Bei den Hairless-Mäusen war bei keiner der Vaccinen 1 bis 5 eine lokale Reaktion und auch keine allgemeine Impfreaktion feststellbar. Die Vaccinen 6 und 7 erzeugten deutliche Impfknoten, die bei 6 nach 14 Tagen nicht mehr nachweisbar oder bei Vaccine 7 nach 21 Tagen noch sichtbar waren.

Bei den Meerschweinchen zeigten die Vaccine 1, 4 und 5 eine kurzzeitige entzündliche Reaktion. Bei Vaccine 6 und 7 war diese Reaktion erheblich stärker ausgeprägt und bei Vaccine 7 21 Tage p.v. (post vaccinationem) noch sichtbar.

Prüfung auf Wirksamkeit:

Mit jeder Vaccine wurden 10 NMRI-Mäuse, 13 - 15 g schwer, mit 0.2 ml pro Tier s.c. vacciniert. 3 Wochen p.v. wurden die Tiere mit gleicher Dosis revacciniert. 1 Woche nach Revaccination wurden die Tiere entblutet und das Serum gewonnen. Im Serum der Tiere waren folgende neutralisierenden Antikörpertiter nachweisbar.

## Tabelle 2:

## Titer neutralisierender Antikörper gegen IBR Virus

| Vaccine | vor Vaccination | 1 Woche nach Revaccination |
|---------|-----------------|----------------------------|
| 1 | kleiner als 1 : 2 | 1 : 224 |
| 2 | kleiner als 1 : 2 | 1 : 12 |
| 3 | - | 1 : 10 |
| 4 | - | 1 : 549 |
| 5 | - | 1 : 198 |
| 6 | kleiner als 1 : 2 | 1 : 697 |
| 7 | - | 1 : 26 |

Dieses Beispiel demonstriert klar, daß alle getesteten Ester in Mäusen und Meerschweinchen verträglicher sind als $Al(OH)_3$. In Mäusen sind alle getesteten Ester auch wirksamer als $Al(OH)_3$. Durch die Kombination Ester + Saponin wird die Wirksamkeit der Vaccine erheblich gesteigert (vergleiche Vaccine 1 und Vaccine 6).

Beispiel 4

Mit inaktiviertem IBR-Antigen wurden 3 Vaccinen hergestellt und an Rindern auf Verträglichkeit und Wirksamkeit geprüft. Vaccine A (Kontrolle) enthielt:

Saponin 10 ml/100 ml    0.5
IBR-Antigen    99.5

Vaccine B enthielt:
Hexansäurehexylester    5.0
[R]Tween 81    1.0
Saponin 10 ml/100 ml    0.5
Entschäumer Bayer E61 E 1881/11    0.5
IBR-Antigen    93.0

Vaccine C enthielt:

Decansäuredodecylester    5.0
[R]Tween 81    1.0
Saponin, 10 ml/100 ml    0.5
Entschäumer Bayer E61 1881/11    0.5
IBR-Antigen    93.0

Mit jeder Vaccine wurden je 2 anti-IBR-Antigen-freie Rinder mit 5.0 ml im Abstand von 3 Wochen 2 ˣ s.c. vacciniert. 1 Woche nach Revaccination wurde von den Tieren eine Blutprobe entnommen und im Serum die neutralisierenden Antikörper gegen das IBR-Virus bestimmt. Dabei wurden die nachfolgenden Antikörpertiter ermittelt.

## Tabelle 3:

## Titer neutralisierender Antikörper gegen IBR-Virus

| Vaccine | Antikörpertiter |
|---------|-----------------|
| A | 1 : 257 |
| | 1 : 382 |
| B | 1 : 876 |
| | 1 : 1149 |
| C | 1 : 994 |
| | 1 : 2044 |

Die Verträglichkeit aller 3 Vaccinen war gut. Die Antikörpertiter der Vaccinen B und C sind deutlich höher als bei Vaccine A.

Beispiel 5

Aus inaktiviertem IBR-Antigen wurden Vaccinen hergestellt Vaccine 1:
Lecithin, 20 g/100 ml Suspension       10.0
IBR-Antigen        90.0

Vaccine 2:
Decansäuredodecylester       5.0
Lecithin, 20 g/100 ml Suspension       10.0
IBR-Antigen       85.00

Vaccine 3:
Decansäuredodecylester       5.0
Al(OH)$_3$, 2 g/100 ml Suspension       5.0
IBR-Antigen        90.0

Vaccine 4:
Al(OH)$_3$, 2 g/100 ml Suspension       5.0
Lecithin, 20 g/100 ml Suspension       10.0
IBR-Antigen        85.0

Mit jeder der 4 Vaccinen wurden je 2 Meerschweinchen mit 0.5 ml s.c. vacciniert. Die Verträglichkeit aller Vaccinen war gut. Lokalreaktionen waren 10 Tage p.v. nicht mehr testbar. Die Wirksamkeit wurde durch die Bestimmung der neutralisierenden Antikörper im Serum der vaccinierten Tiere nachgewiesen (Tab. 2).

## Tabelle 4:

Titer neutralisierender Antikörper

| Vaccine Nr. | vor Vaccination | 3 Woche p. v. |
|---|---|---|
| 1 | kleiner als 1 : 2 | 1 : 12 |
| | kleiner als 1 : 2 | 1 : 28 |
| 2 | 1 : 1 | 1 : 97 |
| | 1 : 2 | 1 : 85 |
| 3 | 1 : 2 | 1 : 78 |
| | 1 : 2 | 1 : 52 |
| 4 | 1 : 2 | 1 : 27 |
| | 1 : 2 | 1 : 14 |

Hierbei zeigt sich klar, daß die Decansäuredodecylsäureester-haltigen Vaccinen höheren Antikörper-Schutz induzieren (Vaccine 2 + 3) als die Al(OH)$_3$ und/oder lecithinhaltigen Vaccinen.

Beispiel 6

Es wurde wie im Beispiel 1 vorgegangen; anstelle von IBR-Antigen wurde inaktiviertes PI$_3$-Antigen eingesetzt. Vaccine 1:
Decansäuredodecylester    4.0
$^R$Tween 81 Emulgator    1.4
Entschäumer Bayer E61E/11    0.5
PI$_3$-Antigen    94.5

Vaccine 2:
$^R$Tween 81    1.0
Entschäumer Bayer E61E/11    0.5
PI$_3$-Antigen    98.5

Vaccine 3:
PI$_3$-Antigen    100.0

Vaccine 4:
Palmitinsäurehexadecylester    1.5
$^R$Tween 81    0.5
PI$_3$-Antigen    98.0

Vaccine 5:
Octansäureoxtylester    5.0
$^R$Tween 81    1.0
Entschäumer Bayer E61E 1811/11    0.5
PI$_3$-Antigen    94.5

Vaccine 6:
Decansäuredodecylester    5.0

$^R$Tween 81     1.0
Entschäumer Bayer E61E 1881/11     0.5
PI$_3$-Antigen     93.0
Saponin 10 ml/100 ml Lösung     0.5

Vaccine 7:
Al(OH)$_3$ 2 g/100 ml Suspension     39.0
PI$_3$-Antigen     61.0

Prüfung auf Wirksamkeit:

Mit jeder Vaccine wurden 10 MRNI-Mäuse, 13-15 g schwer, mit 0.2 ml pro Tier s.c. vacciniert. 3 Wochen p.v. wurden die Tiere mit gleicher Dosis revacciniert. 1 Woche nach Revaccination wurden die Tiere entblutet und das Serum gewonnen. Im Serum der Tiere waren folgende neutralisierende Antikörpertieter nachweisbar.

## Tabelle 5:

## Titer neutralisierender Antikörper gegen PI$_3$ Virus

| Vaccine | vor Vaccination | 1 Woche nach Revaccination |
|---------|-----------------|----------------------------|
| 1 | kleiner als 1:2 | 1:170 |
| 2 | kleiner als 1:2 | 1: 24 |
| 3 | kleiner als 1:2 | 1: 14 |
| 4 | kleiner als 1:2 | 1:389 |
| 5 | kleiner als 1:2 | 1:215 |
| 6 | kleiner als 1:2 | 1:497 |
| 7 | kleiner als 1:2 | 1:110 |

Dieses Beispiel demonstriert klar, daß alle getesteten Ester in Mäusen wirksamer als Al(OH)$_3$ sind. Durch die Kombination Ester + Saponin wird die Wirksamkeit der Vaccine erheblich gesteigert (vergleiche Vaccine 1 und Vaccine 6).

## Ansprüche

1. Alkansäurealkylester der Formel I

R$^1$-CO-O-R$^2$     I

worin
R$^1$ eine lineare Alkylgruppe mit 5, 7, 9, 11, 13, 15 oder 17 Kohlenstoffatomen und
R$^2$ eine lineare Alkylgruppe mit 6, 8, 10, 12, 14, 16 oder 18 Kohlenstoffatomen darstellen.
2. Verfahren zur Herstellung einer Verbindung der Formel I

R$^1$-CO-O-R$^2$     I

worin

$R^1$ eine lineare Alkylgruppe mit 5, 7, 9, 11, 13, 15 oder 17 Kohlenstoffatomen und
$R^2$ eine lineare Alkylgruppe mit 6, 8, 10, 12, 14, 16 oder 18 Kohlenstoffatomen darstellen,
dadurch gekennzeichnet, daß eine Verbindung der Formel II

$R^1$-CO  X    II

worin

X ein die CO-Gruppe aktivierendes Atom oder aktivierende Gruppe ist und
$R^1$ die zur Formel I angegebene Bedeutung hat, mit einer Verbindung der Formel III

$R^2$OH    III

worin $R^2$ die zur Formel I angegebene Bedeutung hat, zu einer Verbindung der Formel I umgesetzt wird.

3. Verwendung einer Verbindung nach Anspruch 1 zur Immunstimulation.

4. Verwendung einer Verbindung nach Anspruch 1 zur Adjuvierung von Vaccinen.

Patentanspruch für folgende Vertragsstaaten : ES und GR:

Verfahren zur Herstellung einer Verbindung der Formel I

$R^1$-CO-O-$R^2$    I

worin

$R^1$ eine lineare Alkylgruppe mit 5, 7, 9, 11, 13, 15 oder 17 Kohlenstoffatomen und
$R^2$ eine lineare Alkylgruppe mit 6, 8, 10, 12, 14, 16 oder 18 Kohlenstoffatomen darstellen,
dadurch gekennzeichnet, daß eine Verbindung der Formel II

$R^1$ CO  X    II

worin

X ein die CO-Gruppe aktivierendes Atom oder aktivierende Gruppe ist und
$R^1$ die zur Formel I angegebene Bedeutung hat, mit einer Verbindung der Formel III

$R^2$OH    III

worin $R^2$ die zur Formel I angegebene Bedeutung hat, zu einer Verbindung der Formel I umgesetzt wird.

Europäisches
Patentamt

**EUROPÄISCHER RECHERCHENBERICHT**

Nummer der Anmeldung

EP 88 10 4696

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | BEILSTEINS HANDBUCH DER ORGANISCHEN CHEMIE, 4. Auflage, 1979, Gesamtregister, Sachregister für die Bände 2,3, Springer-Verlag, Berlin; * Seite 221, Spalte 1, Zeile 54; Spalte 2, Zeilen 3,5,18; Seite 442, Spalte 2, Zeile 53; Seite 443, Spalte 1, Zeilen 19,24,56; Spalte 2, Zeile 3; Seite 516, Spalte 1, Zeilen 3,4,6,7,13,14; Seite 585, Spalte 1, Zeilen 28,30,32,44; Seite 629, Spalte 2, Zeilen 10,25,46,47; Seite 663, Spalte 1, Zeilen 34,57; Spalte 2, Zeile 5; Seite 664, Spalte 1, Zeilen 5,6,15; Seite 803, Spalte 2, Zeilen 17,38,49,53; Seite 804, Spalte 1, Zeilen 54,58; Spalte 2, Zeile 12 * --- | 1 | C 07 C 69/24 A 61 K 47/00 A 61 K 31/23 |
| X | CHEMICAL ABSTRACTS, Band 95, Nr. 7, 17. August 1981, Seite 385, Zusammenfassung Nr. 58384f, Columbus, Ohio, US; A. FERNANDES et al.: "Chemistry of the Dufour's gland secretions of North American andrenid bees (Hymenoptera:Andrenidae)", & J. CHEM. ECOL., 1981 7(2), 453-463, & CHEMICAL ABSTRACTS, 10th Collective Index Ver. 1982, Seite 25315CS, Spalte 1, Zeilen 113,114; Seite 35504CS, Spalte 2. Zeilen 75-77; Seite 35505CS, Spalte 2, Zeilen 101-104; Seite 35508CS, Spalte 2, Zeilen 125-129 --- -/- | 1 | RECHERCHIERTE SACHGEBIETE (Int. Cl.4) C 07 C 69/00 A 61 K 47/00 A 61 K 31/00 |

Der vorliegende Recherchenbericht wurde für alle Patentansprüche erstellt

| Recherchenort | Abschlußdatum der Recherche | Prüfer |
|---|---|---|
| BERLIN | 24-06-1988 | PROBERT C.L. |

Europäisches
Patentamt

**EUROPÄISCHER RECHERCHENBERICHT**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | CHEMICAL ABSTRACTS, Band 93, Nr. 2, 14. Juli 1980, Seite 722, Zusammenfassung Nr. 18608j, Columbus, Ohio, US; H. HENKE et al.: "HPLC of fatty acid esters of mono-and polyhydric alcohols. Part 1. Analytical separation", & HRC CC, J. HIGH RESOLUT. CHROMATOGR. CHROMATOGR. COMMUN. 1980, 3(2), 69-78, & CHEMICAL ABSTRACTS, 10th Collective Index VER. 1982, Seite 25002CS, Spalte 1, Zeilen 51-55<br>--- | 1 | |
| X | CHEMICAL ABSTRACTS, Band 91, Nr. 23, 3. Dezember 1979, Seite 376, Zusammenfassung Nr. 190204u, Columbus, Ohio, US; T.O. GROENNEBERG: "Analysis of a wax ester fraction from the anal gland secretion of beaver (Castor fiber) by chemical ionization mass spectrometry", & CHEM. SCR. 1979, 13(2-3), 56-58, & CHEMICAL ABSTRACTS, 10th Collective Index Ver. 1982, Seite 19355CS, Spalte 2, Zeilen 51-53; Seite 25313CS, Spalten 39-41<br>---          -/- | 1 | RECHERCHIERTE SACHGEBIETE (Int. Cl.4) |

Der vorliegende Recherchenbericht wurde für alle Patentansprüche erstellt

| Recherchenort | Abschlußdatum der Recherche | Prüfer |
|---|---|---|
| BERLIN | 24-06-1988 | PROBERT C.L. |

EPO FORM 1503 03.82 (P0403)

Europäisches
Patentamt

**EUROPÄISCHER RECHERCHENBERICHT**

Nummer der Anmeldung

EP 88 10 4696

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | CHEMICAL ABSTRACTS, Band 79, Nr. 13, 1. Oktober 1973, Seite 423, Zusammenfassung Nr. 77900g, Columbus, Ohio, US; L. MAROSI et al.: "Long-chain carboxylic acid esters. Melting points, rules for the occurrence of the vertical crystal structure type, and increments of long interplanar spacings", & JUSTUS LIEBIGS ANN. CHEM. 1973, (4), 584-598, & CHEMICAL ABSTRACTS, 9th Collective Index Ver. 1978, Seite 13318CS, Spalte 1, Zeilen 20-23; Seite 13319CS, Spalte 3, Zeilen 111-115; Seite 13320CS, Spalte 3, Zeilen 110-111; Seite 13321CS, Spalte 1, Zeilen 1,2; Seite 12944F, Spalte 3, Zeilen 55,56 --- | 1 | |
| X | J. MARCH: "Avanced Organic Chemistry", 2. Edition 1977, Seiten 361-367, McGraw-Hill Verlag, London, GB; Seiten 361-367 --- | 1,2 | **RECHERCHIERTE SACHGEBIETE (Int. Cl.4)** |
| A | EP-A-0 131 790 (TROPON WERKE) * Seite 3, Zeile 19 - Seite 4, Zeile 3 * --- | 3,4 | |
| A | EP-A-0 108 316 (BAYER) * Seite 5, Zeilen 13-24 * --- | 3,4 | |
| A | US-A-4 171 353 (RYAN) * Ansprüche * --- | 3,4 | |
| A | US-A-4 058 594 (WILLIAMS) * Ansprüche * --- -/- | 3,4 | |

Der vorliegende Recherchenbericht wurde für alle Patentansprüche erstellt

| Recherchenort | Abschlußdatum der Recherche | Prüfer |
|---|---|---|
| BERLIN | 24-06-1988 | PROBERT C.L. |

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | US-A-4 049 824   (DIEHL) <br> * Ansprüche * <br> --- | 3,4 | |
| A | EP-A-0 059 521   (CENTRAAL DIERGENESSKUNDIG INSTITUUT) <br> * Anspruch 5 * <br> ----- | 3 | |

RECHERCHIERTE
SACHGEBIETE (Int. Cl.4)

Der vorliegende Recherchenbericht wurde für alle Patentansprüche erstellt

| Recherchenort | Abschlußdatum der Recherche | Prüfer |
|---|---|---|
| BERLIN | 24-06-1988 | PROBERT C.L. |